(19) Europäisches Patentamt
European Patent Office
Office européen des brevets

(11) Veröffentlichungsnummer: **0 187 618**
**A1**

# (12) EUROPÄISCHE PATENTANMELDUNG

(21) Anmeldenummer: 85810564.6

(22) Anmeldetag: 27.11.85

(51) Int. Cl.[4]: C 07 D 277/56
A 61 K 31/425

(30) Priorität: 30.11.84 DE 3443698
12.06.85 DE 3520976

(43) Veröffentlichungstag der Anmeldung:
16.07.86 Patentblatt 86/29

(84) Benannte Vertragsstaaten:
AT BE CH DE FR GB IT LI LU NL SE

(71) Anmelder: SANDOZ AG
Lichtstrasse 35
CH-4002 Basel(CH)

(84) Benannte Vertragsstaaten:
BE CH FR GB IT LI LU NL SE

(71) Anmelder: SANDOZ-PATENT-GMBH
Humboldtstrasse 3
D-7850 Lörrach(DE)

(84) Benannte Vertragsstaaten:
DE

(71) Anmelder: SANDOZ-ERFINDUNGEN
Verwaltungsgesellschaft m.b.H.
Brunner Strasse 59
A-1235 Wien(AT)

(84) Benannte Vertragsstaaten:
AT

(72) Erfinder: Nesvadba, Hans
Doeblinger Hauptstrasse 1
A-1190 Wien(AT)

(72) Erfinder: Besemer-Rosenwirth, Brigitte
Carl Zwillinggasse 17/2
A-2340 Mödling(AT)

## (54) Piperazinderivate, Verfahren zu ihrer Herstellung und ihre Verwendung.

(57) Die Erfindung betrifft neue Piperazinderivate der Formel

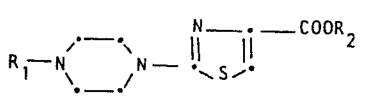

I

worin $R_1$ für Wasserstoff oder eine Gruppe der Formel

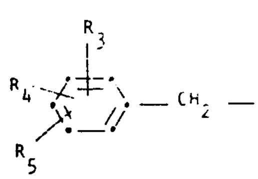

II

wobei $R_3$, $R_4$ und $R_5$ gleich oder verschieden sein können und jeweils Wasserstoff, eine niedere Alkyl-, eine niedere Alkoxygruppe, $CF_3$, $CCl_3$, $CHF_2$, CN, $NO_2$ oder Halogen bedeuten, und $R_2$ für Wasserstoff oder eine niedere Alkylgruppe stehen, und ihre Säureadditionssalze sowie Verfahren zur Herstellung der Verbindungen der Formel I und ihrer Säureadditionssalze, und ihre Verwendung als antivirale Mittel

EP 0 187 618 A1

Croydon Printing Company Ltd

Piperazinderivate, Verfahren zu ihrer Herstellung und ihre Verwendung

Die Erfindung betrifft neue Piperazinderivate der Formel

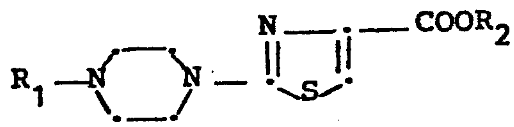

I

worin $R_1$ für Wasserstoff oder eine Gruppe der Formel

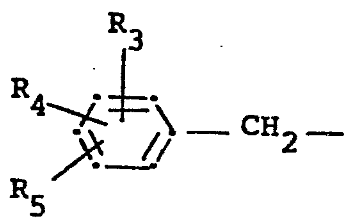

II

wobei $R_3$, $R_4$ und $R_5$ gleich oder verschieden sein können und jeweils Wasserstoff, eine niedere Alkyl-, eine niedere Alkoxy-, eine niedere Halogenalkylgruppe, CN, $NO_2$ oder Halogen bedeuten, und $R_2$ für Wasserstoff oder eine niedere Alkylgruppe stehen, und ihre Säureadditionssalze und ihre Basensalze.

Erfindungsgemäß gelangt man zu den Verbindungen der Formel I und zu ihren Säureadditionssalzen und Basensalzen, indem man

a) zur Herstellung von Verbindungen der Formel

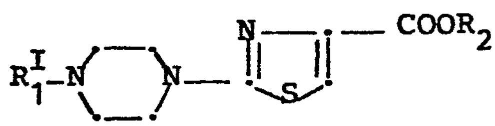

Ia

worin $R_2$ obige Bedeutung besitzt und $R_1^I$ die gleiche Bedeutung wie $R_1$ mit Ausnahme von Wasserstoff hat, eine Verbindung der Formel

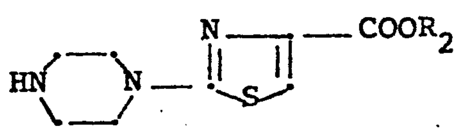

Ib

worin $R_2$ obige Bedeutung besitzt, mit einer Verbindung der Formel

$$R_1^I - X \qquad III$$

worin X eine Abgangsgruppe bedeutet und $R_1^I$ obige Bedeutung besitzt, umsetzt, oder

b) zur Herstellung von Verbindungen der Formel I eine Verbindung der Formel

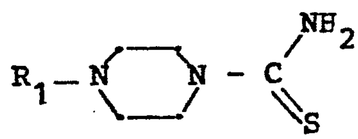

IV

worin $R_1$ obige Bedeutung besitzt, mit einer Verbindung der Formel

$$Y\text{-}CH_2.CO.COOR_2 \qquad V$$

worin Y eine Abgangsgruppe bedeutet und $R_2$ obige Bedeutung besitzt, umsetzt, oder

c) eine Verbindung der Formel

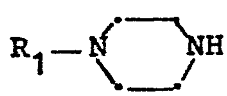

VI

worin $R_1$ obige Bedeutung besitzt, mit einer Verbindung der Formel

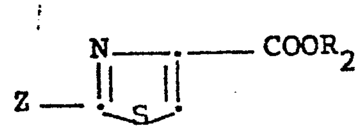

VII

worin Z für eine Abgangsgruppe steht und $R_2$ obige Bedeutung besitzt, umsetzt und gewünschtenfalls Verbindungen der Formel I, worin $R_2$ für eine niedere Alkylgruppe steht, anschließend in Verbindungen der Formel I, worin $R_2$ für Wasserstoff steht, überführt und/oder gegebenenfalls Verbindungen der Formel I in ihre Säureadditionssalze oder Basensalze überführt.

Verbindungen der Formel Ia können nach Verfahren a) beispielsweise erhalten werden, indem man eine Verbindung der Formel Ib in einem unter den Reaktionsbedingungen inerten Lösungsmittel, beispielsweise in einem Dialkylcarbonsäureamid, wie Dimethylformamid, löst und mit einer Verbindung der Formel III zur Reaktion bringt, vorzugsweise unter Zusatz eines Säurebindemittels wie $K_2CO_3$.

Die Cyclisierung nach Verfahren b) kann beispielsweise ausgeführt werden, indem man eine Verbindung der Formel IV in einem unter den Reaktionsbedingungen inerten Lösungsmittel, z.B. in einem wasserfreien niederen Alkohol, wie absolutem Ethanol, löst bzw. suspendiert und mit einer Verbindung der Formel V, z.B. mit Brombrenztraubensäureester vorzugsweise bei erhöhter Temperatur, z.B. bei Siedetemperatur des Reaktionsgemisches, reagieren läßt.

Zur Durchführunng der Verfahrensvariante c) kann man beispielsweise eine Verbindung der Formel VI in einem unter den Reaktionsbedingungen inerten Lösungsmittel, z. B. in einem cyclischen Ether, wie Tetrahydrofuran, mit einer Verbindung der Formel VII versetzen und vorzugsweise bei erhöhter Temperatur, z. B. bei Siedetemperatur des Reaktionsgemisches, reagieren lassen.

Aus dem Reaktionsgemisch können die Endprodukte nach bekannten Methoden isoliert und gegebenenfalls gereinigt werden.

Die als Substituenten aufscheinenden bzw. in Substituenten enthaltenen niederen Alkylgruppen besitzen vorzugsweise 1 bis 4 Kohlenstoffatome und bedeuten insbesondere Methyl oder Ethyl. Niederes Halogenalkyl bedeutet

vorzugsweise $CF_3$, $CHF_2$ oder $CCl_3$, insbesondere $CF_3$. Als Abgangsgruppen X, Y und Z können die bei diesen bekannten Verfahren üblicherweise eingesetzten Gruppen verwendet werden, beispielsweise Halogen, vorzugsweise Chlor oder Brom.

Die Ausgangsprodukte der Formel IV, worin $R_1$ nicht für Wasserstoff steht, sind teilweise neu und können bespielsweise nach folgendem Reaktionsschema erhalten werden:

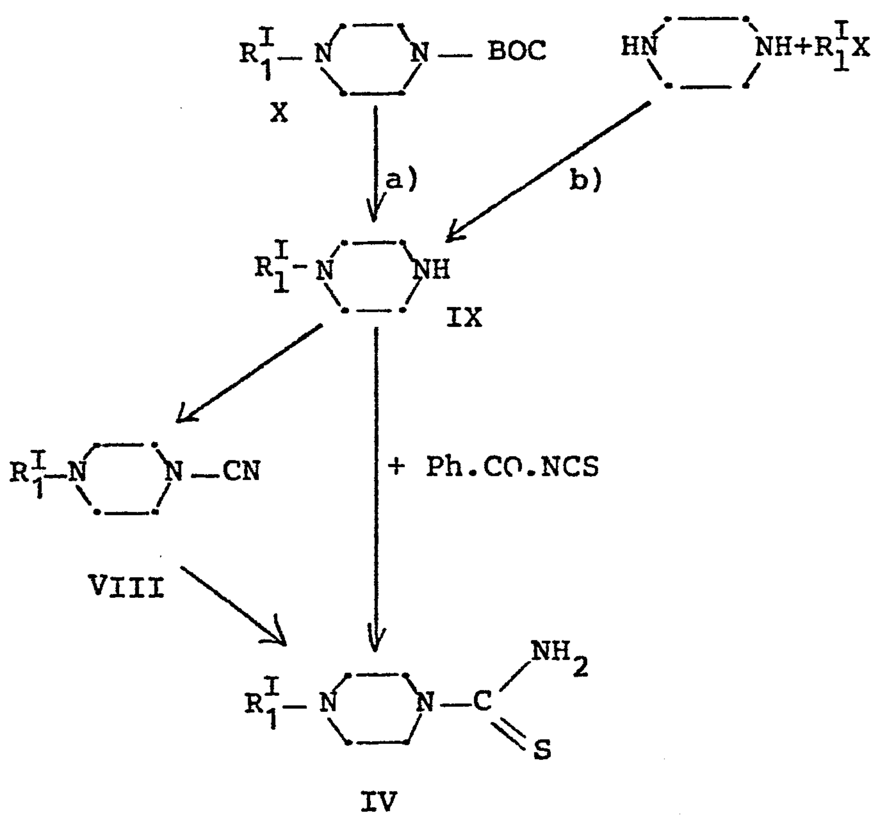


Bei der Herstellung der Verbindungen der Formel IX nach Variante b) wird das Piperazinhydrat im Überschuß eingesetzt, beispielsweise in 5- bis 10-facher, vorzugsweise in 7- bis 8-facher Menge, bezogen auf $R_1^I$-X.

Die übrigen Ausgangsprodukte sind bekannt oder nach bekannten Verfahren bzw. analog zu bekannten Verfahren herstellbar.

Die Verbindungen der Formel I können durch Umsetzung mit geeigneten anorganischen oder organischen Säuren in ihre Säureadditionssalze übergeführt werden. Hierfür sind als anorganische Säuren, beispielsweise Halogenwasserstoffsäuren, und als organische Säuren, beispielsweise Maleinsäure, geeignet.

Die Verbindungen der Formel I zeigen vorteilhafte pharmakologische, insbesondere antivirale Eigenschaften, wie durch ihre Hemmwirkung gegen verschiedene Viren, z.B. Picornaviren, insbesondere gegen Rhinoviren gezeigt werden konnte. Diese Hemmwirkung wurde in vitro bei verschiedenen Konzentrationen, z.B. ab 0,0001 bis 100 µg/ml nachgewiesen. Die Verbindungen können daher als antivirale Substanzen verwendet werden, z.B. zur Bekämpfung von virusinduzierten respiratorischen Erkrankungen.

Für diese Verwendung beträgt eine tägliche Dosis ca. 70 bis 350 mg. Diese Menge kann gegebenenfalls in entsprechend kleineren Dosen zwei- bis viermal täglich oder in Retardform gegeben werden. Für orale Verabreichung geeignete Dosierungsformen beinhalten 17,5 bis 350 mg an aktiver Substanz, vermischt mit einem festen oder flüssigen pharmazeutischen Träger.

Die Verbindungen der Formel I, worin $R_1$ für Wasserstoff steht, können in Form der freien Basen oder in Form pharmazeutisch unbedenklicher Säureadditionssalze verwendet werden, wobei die Salze größenordnungsmäßig die gleiche Wirksamkeit besitzen wie die entsprechenden freien Basen. Geeignete Säureadditionssalze sind z.B. die Hydrochloride, Hydrogenfumarate und Naphthalin-1,5-disulfonate.

Die Verbindungen der Formel I, worin $R_2$ für Wasserstoff steht, können auch in Form pharmazeutisch akzeptabler Basensalze, z. B. des Natrium- oder Kaliumsalzes, eingesetzt werden.

Die Verbindungen der Formel I können oral, topical, intravnös oder parenteral verabreicht werden.

Als Heilmittel können die Verbindungen der Formel I und gegebenenfalls ihre wasserlöslichen, physiologisch verträglichen Salze allein oder in geeigneten Arzneiformen gemeinsam mit anorganischen oder organischen, pharmakologisch indifferenten Hilfsstoffen verabreicht werden. Beispielsweise werden sie als Bestandteil von Injektions- oder Instillationszubereitungen, von Kapseln, Trinklösungen, Sprays, Salben, Aerosols, Nasentropfen usw. eingesetzt.

Die Erfindung betrifft auch eine Verbindung der Formel I zur Verwendung als Pharmazeutikum, insbesondere als antivirales Mittel.

Ein weiterer Aspekt dieser Erfindung betrifft eine Methode zur Behandlung von viralen Erkrankungen durch Verabreichung einer effektiven Menge einer Verbindung der Formel I an den Kranken.

Außerdem betrifft die Erfindung eine pharmazeutische Zusammensetzung, bestehend aus einer Verbindung der Formel I und einem pharmazeutisch akzeptablen Verdünnungsmittel oder Trägerstoff.

Besondere Bedeutungen der Substituenten sind:

$R_1$ = a) Formel II
b) Benzyl
c) Mono-, Di- oder Trialkylbenzyl

$R_2$ = a) Wasserstoff
b) $(C_{1-4})$Alkyl

Kombinationen davon sind besonders bevorzugt.

Beispiele bestimmter Verbindungsgruppen sind folgende:

$R_1$ bedeutet eine Gruppe der Formel II, worin $R_3$, $R_4$ und $R_5$ unabhängig voneinander niederes Alkyl bedeuten, und

$R_2$ besitzt obige Bedeutung, oder

$R_1$ bedeutet Wasserstoff oder eine Gruppe der Formel II, wobei $R_4$ Wasserstoff, niederes Alkyl, niederes Alkoxy, $CF_3$ oder Halogen bedeutet

$R_5$ bedeutet Wasserstoff, niederes Alkyl oder niederes Alkoxy und

$R_3$ und $R_2$ besitzen obige Bedeutung, oder

$R_1$ steht für Wasserstoff oder eine Gruppe der Formel

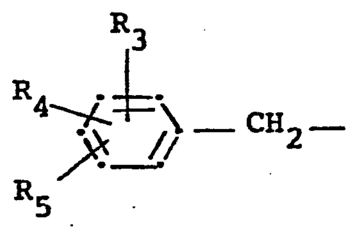

II

worin $R_3$, $R_4$ und $R_5$ Wasserstoff, niederes Alkyl, niederes Alkoxy, $CF_3$, $NO_2$ oder Halogen bedeuten und

$R_2$ für Wasserstoff oder niederes Alkyl steht.

Eine besonders bevorzugte Verbindung ist die 2-[1-(2,5-Dimethylbenzyl)piperazin-4-yl]-1,3-thiazol-4-carbonsäure in freier Form, in Form des Natriumsalzes oder als Ethylester.

In den nachfolgenden Beispielen, die die Erfindung näher erläutern, ihren Umfang jedoch in keiner Weise einschränken sollen, erfolgen alle Temperaturangaben in Celsiusgraden.

Beispiel 1: 2-[1-(2,5-Dimethylbenzyl)piperazin-4-yl]-1,3-thiazol-4-carbonsäureethylester (Verfahren b):

140 g 1-(2,5-Dimethylbenzyl)piperazin-4-carbothioamid werden in 2000 ml absolutem Ethanol suspendiert, 70 g Brombrenztraubensäureethylester zugegeben und unter Rühren 30 Minuten rückflußgekocht. Ungelöster Feststoff wird heiß abgesaugt, das Filtrat mit Eiswasser gekühlt und abgesaugt. Die Mutterlauge wird eingedampft und der resultierende Rückstand aus Ethanol umkristallisiert. Die gesammelten Kristallisate werden in 1500 ml Chloroform suspendiert, mit einer Lösung von 100 g Kaliumkarbonat in 800 ml Wasser versetzt und 30 Minuten gerührt. Die organische Phase wird einmal mit 50 ml gesättigter NaCl-Lösung ausgeschüttelt, mit Magnesiumsulfat getrocknet und einrotiert. Der Rückstand wird mit 500 ml Petroleumbenzin (40-60°) digeriert, abgesaugt und getrocknet. Die Mutterlauge wird eingedampft, in 200 ml Chloroform gelöst und über 2,5 kg Kieselgel 60 (Korngröße 0,040-0,063 mm) chromatographiert. Die entsprechende Fraktion wird eingedampft und mit dem oben erhaltenen Rückstand vereinigt. Fp: 88-89° (Fp des Hydrochlorids: 210-220°).

Beispiel 2: 2-[1-(2,5-Dimethylbenzyl)piperazin-4-yl]-1,3-thiazol-4-carbonsäure.Natriumsalz:

146 g 2-[1-(2,5-Dimethylbenzyl)piperazin-4-yl]-1,3-thiazol-4-carbonsäureethylester werden in 2500 ml Methanol gelöst, 1000 ml 1 N Natronlauge zugegeben, über Nacht gerührt und einrotiert. Der Rückstand wird in 2 l Wasser gelöst, einmal mit 500 ml Chloroform ausgeschüttelt, die lösungsmittelfreie wäßrige Lösung mit verdünnter Salzsäure (1/1) auf pH = 6 eingestellt, ausgefallene Substanz abgesaugt und getrocknet. Die Mutterlauge wird eingedampft, mit 200 ml 0,5 N Natronlauge gelöst, wieder auf pH = 6 eingestellt, abgesaugt und getrocknet. Die gesammelten Kristallisate werden in 382,2 ml 1 N Natronlauge gelöst und gefriergetrocknet. Fp: ab 180° (Zers.).

Beispiel 3: 2-(1-Piperazinyl)-1,3-thiazol-4-carbonsäureethylester (Verfahren b):

0,25 g Piperazin-1-carbothioamid werden in 10 ml absolutem Ethanol mit

0,36 g Brombrenztraubensäureethylester versetzt und 2 Stunden bei Raumtemperatur stehengelassen. Dann wird im Vakuum auf 1/4 des Volumens eingeengt, mit 10 ml Diethylether versetzt, abfiltriert und im Vakuum bei Raumtemperatur 18 Stunden über NaOH getrocknet. Fp (Dihydrochlorid): 236-239°.

Beispiel 4: 2-(1-Benzylpiperazin-4-yl)-1,3-thiazol-4-carbonsäureethylester (Verfahren a):

0,5 g 2-(1-Piperazinyl)-1,3-thiazol-4-carbonsäureethylester.HBr werden in 10 ml Dimethylformamid gelöst und nach Zusatz von 1 g fein gepulvertem $K_2CO_3$ und 0,2 g Benzylchlorid 24 Stunden bei Raumtemperatur gerührt. Danach wird vom anorganischen Salz filtriert, einrotiert, in 50 ml Essigsäureethylester gelöst, mit Wasser und gesättigter Natriumchloridlösung gewaschen, über $MgSO_4$ getrocknet und im Vakuum einrotiert. Die Substanz kann durch Destillation (1,33 x $10^{-3}$ mbar/170°) gereinigt werden. Fp: 107-109° (Fp des Hydrochlorids: 206-209°).

Beispiel 5: 2-(1-Benzylpiperazin-4-yl)-1,3-thiazol-4-carbonsäureethylester (Verfahren c):

4 g N-Benzylpiperazin werden in 150 ml Tetrahydrofuran gelöst, mit 2,7 g 2-Brom-1,3-thiazol-4-carbonsäureethylester versetzt und 2 Stunden unter Rückfluß gekocht. Man dampft das Lösungsmittel ab, nimmt den Rückstand in 8 %iger $NaHCO_3$-Lösung auf und extrahiert mit Essigester. Die organische Phase wird mit konz. NaCl-Lösung einmal nachgeschüttelt, über Magnesiumsulfat getrocknet und bei 16 mbar einrotiert. Fp: 107-109° (Fp des Hydrochlorids: 206-209°).

Analog wie in den vorhergehenden Beispielen beschrieben, können auch folgende Verbindungen der Formel I erhalten werden:

| Bp: | $R_3$ | $R_4$ | $R_5$ | $R_2$ | Fp (HCl): |
|---|---|---|---|---|---|
| 6 | 2-Cl | H | H | $C_2H_5$ | 213-216° |
| 7 | 4-Cl | H | H | $C_2H_5$ | 196-200° |
| 8 | 2-Cl | 6-Cl | H | $C_2H_5$ | 176-180° |
| 9 | 3-$CF_3$ | H | H | $C_2H_5$ | 186-188° |
| 10 | H | H | H | H | 285-286° |
| 11 | 4-CN | H | H | $C_2H_5$ | 180-184° |
| 12 | 4-$NO_2$ | H | H | $C_2H_5$ | 186-189° |
| 13 | 2-$CH_3$ | H | H | $C_2H_5$ | 192-195° |
| 14 | 3-$CH_3$ | 4-$CH_3$ | 5-$CH_3$ | $C_2H_5$ | 175-180° |
| 15 | 4-Br | H | H | $C_2H_5$ | 205-210° |
| 16 | 4-$C(CH_3)_3$ | H | H | $C_2H_5$ | 212-215° |
| 17 | 3-$OCH_3$ | 4-$OCH_3$ | 5-$OCH_3$ | $C_2H_5$ | 195-198° |
| 18 | 3-$CH_3$ | 5-$CH_3$ | H | $C_2H_5$ | 194-196° |
| 19 | 4-i-Propyl | H | H | $C_2H_5$ | 202-205° |
| 20 | 3-$CH_3$ | H | H | $C_2H_5$ | 196-200° |
| 21 | 3-Cl | 4-Cl | H | $C_2H_5$ | 118-120° |
| 22 | 3-$CH_3$ | 4-$CH_3$ | H | $C_2H_5$ | 196-198° |
| 23 | 2-$CH_3$ | 4-$CH_3$ | H | $C_2H_5$ | 186-189° |
| 24 | 4-$CH_3$ | H | H | $C_2H_5$ | 178-182° |
| 25 | 3-Cl | 5-Cl | H | $C_2H_5$ | 114-118° |
| 26 | 4-$C_2H_5$ | H | H | $C_2H_5$ | 195-197° |
| 27 | 4-F | H | H | $C_2H_5$ | 204-207° |
| 28 | 2-$CH_3$ | 3-$CH_3$ | H | $C_2H_5$ | 178-182° |
| 29 | 2-$OCH_3$ | 3-$OCH_3$ | H | $C_2H_5$ | 165-168° |
| 30 | 2-$CH_3$ | 6-$CH_3$ | H | $C_2H_5$ | 182-185° |
| 31 | 3-$CF_3$ | 5-$CF_3$ | H | $C_2H_5$ | 148-150° |
| 32 | 3-$CHF_2$ | 5-$CF_3$ | H | $C_2H_5$ | 130-133° |
| 33 | 3-$CH_3$ | 5-$CF_3$ | H | $C_2H_5$ | 121-122° |
| 34 | 3-$CF_3$ | 4-Cl | H | $C_2H_5$ | 184-187° |
| 35 | 3-$CCl_3$ | H | H | $C_2H_5$ | 210-212° |
| 36 | 2-$CF_3$ | H | H | $C_2H_5$ | 172-174° |
| 37 | 4-$CF_3$ | H | H | $C_2H_5$ | 181-184° |
| 38 | 2-$CF_3$ | 5-$CF_3$ | H | $C_2H_5$ | 115-118° |
| 39 | 2-$CF_3$ | 4-$CF_3$ | H | $C_2H_5$ | 112-115° |
| 40 | 2-$CF_3$ | 6-$CF_3$ | H | $C_2H_5$ | 134-138° |
| 41 | 2-OCH3 | 5-OCH3 | H | C2H5 | 125-128° |

Das als Ausgangsprodukt benötigte 1-(2,5-Dimethylbenzyl)piperazin-4-carbothioamid kann folgendermaßen erhalten werden:

a) 1-(2,5-Dimethylbenzyl)-4-tert.butyloxycarbonylpiperazin

Man löst 159 g 1-tert.Butyloxycarbonylpiperazin in 1000 ml N,N-Dimethylformamid, gibt 60 g feingepulvertes Kaliumkarbonat zu und tropft unter Eiskühlung 132 g 2,5-Dimethylbenzylchlorid zu. Die Mischung wird über Nacht bei Raumtemperatur gerührt (Gasentwicklung!), vom Salz abgesaugt und am Rotationsverdampfer eingedampft. Der Rückstand wird in 2000 ml Essigsäureethylester/Wasser (3/1) aufgenommen, einmal mit gesättigter NaCl-Lösung ausgeschüttelt, mit Magnesiumsulfat getrocknet und einrotiert. Man erhält die Titelverbindung als gelbliches Öl.

b) 1-(2,5-Dimethylbenzyl)piperazin

260 g 1-(2,5-Dimethylbenzyl)-4-tert.butyloxycarbonylpiperazin werden unter Eiskühlung mit 1500 ml ethanolischer Salzsäure versetzt (heftige Gasentwicklung.), nach einer Stunde Reaktionszeit am Rotationsverdampfer eingedampft, 2000 ml absoluter Diethylether zugesetzt, abgesaugt und getrocknet. Das Kristallisat wird in Dichlormethan aufgenommen und unter Eiskühlung Ammoniakgas bis zur Sättigung eingeleitet. Die entstandene dicke Suspension wird abgesaugt, gut gewaschen und das Filtrat einrotiert. Man erhält die Titelverbindung in Form weißer Kristalle. Fp: 59-63°.

Das 1-(2,5-Dimethylbenzyl)piperazin kann auch direkt aus Piperazin erhalten werden. Zu einer Lösung von 38,2 g Piperazin (wasserfrei) in 100 ml absolutem Ethanol werden unter Rühren bei 10-15° in einem Zeitraum von 30 Minuten 10 g 2,5-Dimethylbenzylchlorid zugetropft. Nach weiteren 3 Stunden Reaktionszeit bei Raumtemperatur wird im Vakuum das Lösungsmittel entfernt, der Rückstand wiederholt mit Diethylether extrahiert und die im Vakuum eingeengten Extrakte über Kieselgel (M 7734, offene Säule, Ø = 5 cm, l = 1 m, Laufmittel: $CHCl_3/CH_3OH/33$ % $NH_3$-Lösung = 85/15/2) chromatographiert. Der Rohextrakt kann auch durch fraktionierte Vakuumdestillation von noch vorhandenem Piperazin getrennt werden.

c) 1-(2,5-Dimethylbenzyl)-4-cyanopiperazin

Man löst 120 g 1-(2,5-Dimethylbenzyl)piperazin in 800 ml N,N-Dimethylformamid, setzt 50 g feingepulvertes Kaliumkarbonat zu und tropft bei Eiskühlung und unter Rühren langsam eine Lösung von 63 g Bromcyan in 200 ml N,N-Dimethylformamid zu. Es wird bei Raumtemperatur über Nacht gerührt, vom anorganischen Salz abgesaugt, bei 0,13 mbar/30° eingedampft und in 2 l Essigsäureethylester/Wasser (3/1) aufgenommen. Die organische Phase wird einmal mit 50 ml gesättigter NaCl-Lösung nachgewaschen, über Magnesiumsulfat getrocknet und einrotiert. Der ölige Rückstand wird anschließend mit Petroleumbenzin (40-60°) digeriert, wobei Kristallisation eintritt. Das resultierende Kristallisat wird mit Petroleum benzin (60-80°) digeriert, abgesaugt und getrocknet. Man erhält die Titelverbindung als leicht gebliches Kristallisat. Fp: 56-60°.

d) 1-(2,5-Dimethylbenzyl)piperazin-4-carbothioamid

In einem Druckgefäß werden in 350 ml Methanol bei -20° 35 g Ammoniak und 70 g Schwefelwasserstoff kondensiert, bei Raumtemperatur 125 g 1-(2,5-Dimethylbenzyl)-4-cyanopiperazin zugegeben und bis zur klaren Lösung geschüttelt. Anderntags wird eingedampft, mit 1000 ml Diethylether versetzt, digeriert, abgesaugt und getrocknet. Man erhält die Titelverbindung als weißes Kristallisat. Fp: 176-180°.

Analog wie hier beschrieben, können auch die übrigen Verbindungen der Formel IV erhalten werden.

Patentansprüche:

1. Piperazinderivate der Formel

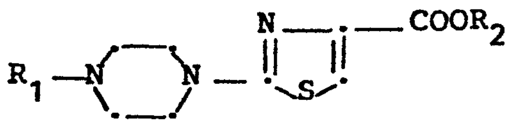

I

worin $R_1$ für Wasserstoff oder eine Gruppe der Formel

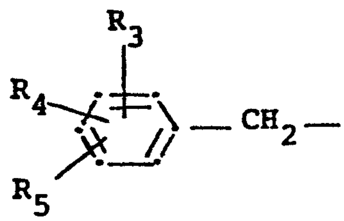

II

wobei $R_3$, $R_4$ und $R_5$ gleich oder verschieden sein können und jeweils Wasserstoff, eine niedere Alkyl-, eine niedere Alkoxy-, eine niedere Halogenalkylgruppe, CN, $NO_2$ oder Halogen bedeuten, und $R_2$ für Wasserstoff oder eine niedere Alkylgruppe stehen, und ihre Säureadditionssalze und Basensalze.

2. Eine Verbindung entsprechend Anspruch 1, worin

a) $R_1$ eine Gruppe der Formel II bedeutet, wobei $R_3$, $R_4$ und $R_5$ unabhängig voneinander für niederes Alkyl stehen, und $R_2$ obige Bedeutung besitzt,

b) $R_1$ Wasserstoff oder eine Gruppe der Formel II bedeutet, wobei $R_4$ für Wasserstoff niederes Alkyl, niederes Alkoxy, $CF_3$ oder Halogen steht, $R_5$ Wasserstoff, niederes Alkyl oder niederes Alkoxy bedeutet und $R_2$ und $R_3$ obige Bedeutung besitzen.

3. Eine Verbindung entsprechend Anspruch 1, worin $R_1$ für Wasserstoff oder eine Gruppe der Formel

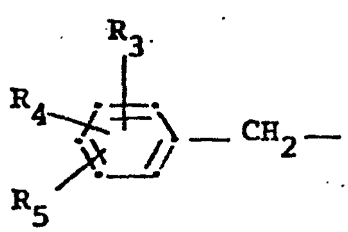

II

wobei $R_3$, $R_4$ und $R_5$ Wasserstoff niederes Alkyl, niederes Alkoxy, $CF_3$, $NO_2$ oder Halogen bedeuten, und $R_2$ für Wasserstoff oder niederes Alkyl stehen.

4. 2-[1-(2,5-Dimethylbenzyl)piperazin-4-yl]-1,3-thiazol-4-carbonsäure.

5. Eine Verbindung entsprechend Anspruch 4) in Form des Natriumsalzes oder des Ethylesters.

6. Eine pharmazeutische Zusammensetzung, die eine Verbindung der Formel I entsprechend Anspruch 1 in freier Form oder in Form eines pharmazeutisch akzeptablen Säureadditionssalzes oder Basensalzes, zusammen mit einem pharmazeutischen Verdünnungsmittel oder Trägerstoff enthält.

7. Eine Verbindung der Formel I, entsprechend Anspruch 1, in freier Form oder in Form eines pharmazeutisch akzeptablen Säureadditionssalzes oder Basensalzes, zur Verwendung zur Herstellung eines Medikaments.

8. Eine Verbindung der Formel I, entsprechend Anspruch 1, in freier Form oder in Form eines pharmazeutisch akzeptablen Säureadditionssalzes oder Basensalzes, zur Verwendung zur Herstellung eines antiviralen Medikaments.

9. Verfahren zur Herstellung neuer Piperazinderivate der Formel

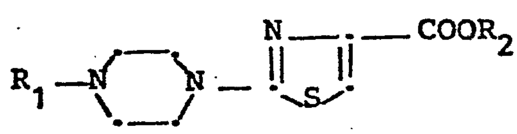

I

worin $R_1$ für Wasserstoff oder eine Gruppe der Formel

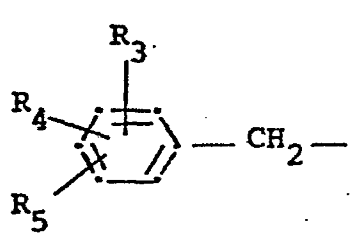

II

wobei $R_3$, $R_4$ und $R_5$ gleich oder verschieden sein können und jeweils Wasserstoff, eine niedere Alkyl-, eine niedere Alkoxy-, eine niedere Halogenalkylgruppe, CN, $NO_2$ oder Halogen bedeuten, und $R_2$ für Wasserstoff oder eine niedere Alkylgruppe stehen, und ihrer Säureadditionssalze und Basensalze, dadurch gekennzeichnet, daß man

a) zur Herstellung von Verbindungen der Formel

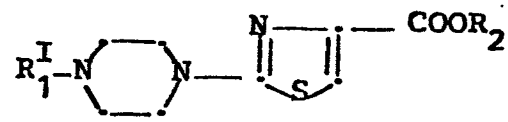

Ia

worin $R_2$ obige Bedeutung besitzt und $R_1^I$ die gleiche Bedeutung wie $R_1$ mit Ausnahme von Wasserstoff hat, eine Verbindung der Formel

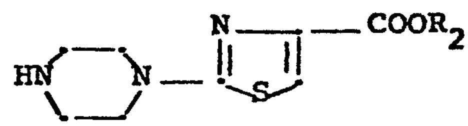

Ib

worin $R_2$ obige Bedeutung besitzt, mit einer Verbindung der Formel

$$R_1^I - X \qquad \text{III}$$

worin X eine Abgangsgruppe bedeutet und $R_1^I$ obige Bedeutung besitzt, umsetzt, oder

b) zur Herstellung von Verbindungen der Formel I eine Verbindung der Formel

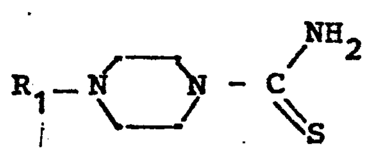

IV

worin $R_1$ obige Bedeutung besitzt, mit einer Verbindung der Formel

$$Y\text{-}CH_2.CO.COOR_2 \qquad V$$

worin Y eine Abgangsgruppe bedeutet und $R_2$ obige Bedeutung besitzt, umsetzt, oder

c) eine Verbindung der Formel

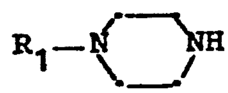

VI

worin $R_1$ obige Bedeutung besitzt, mit einer Verbindung der Formel

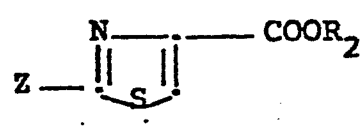

VII

worin Z für eine Abgangsgruppe steht und $R_2$ obige Bedeutung besitzt, umsetzt und gewünschtenfalls Verbindungen der Formel I, worin $R_2$ für eine niedere Alkylgruppe steht, anschließend in Verbindungen der Formel I, worin $R_2$ für Wasserstoff steht, überführt und/oder gegebenenfalls Verbindungen der Formel I in ihre Säureadditionssalze oder Basensalze überführt.

10. Eine Verbindung der Formel

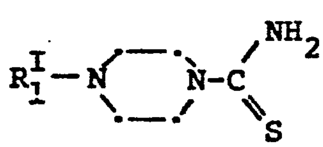

IVa

worin $R_1^I$ obige Bedeutung besitzt.

Europäisches Patentamt

# EUROPÄISCHER RECHERCHENBERICHT

0187618

Nummer der Anmeldung

EP 85 81 0564

| Kategorie | EINSCHLÄGIGE DOKUMENTE — Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | GB-A-2 000 501 (SCIENCE UNION)<br>--- | | C 07 D 277/56<br>A 61 K 31/425 |
| A | GB-A-1 462 350 (WANDER)<br>--- | | |
| A | GB-A-1 518 559 (SCIENCE UNION)<br>--- | | |
| A | EP-A-0 005 091 (SCIENCE UNION)<br>----- | | |
| | | | RECHERCHIERTE SACHGEBIETE (Int Cl.4) |
| | | | C 07 D 277/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 05-03-1986 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82